# EUROPEAN PATENT APPLICATION

(11) **EP 4 497 406 A1**
(43) Date of publication of application: **29.01.2025**
(21) Application number: 23306291.8
(22) Date of filing: 25.07.2023
(51) Int. Cl.: A61B 34/30

(54) **SURGICAL ROBOTIC SYSTEM AND METHOD FOR DEFINING A RESTRICTED ACCESS VOLUME FOR SUCH A SURGICAL ROBOTIC SYSTEM**

(71) Applicant: Ecential Robotics, 38610 Gieres (FR)
(72) Inventor: VIDAL, Clément, 38610 GIERES (FR); SIEFFERT, Jérôme, 38610 GIERES (FR); RAHAL, Rahaf, 38610 GIERES (FR)
(74) Representative: Regimbeau

(57) **Abstract**

The invention relates to a method for determining a restricted access volume for a surgical robotic system comprising a robotic arm and an end-effector, a registration phantom comprising a set of radiopaque fiducials, the registration phantom being located in proximity to the region of interest, the method comprising the following steps: acquiring at least one 2D X-ray image containing the region of interest and the set of fiducials of the registration phantom by an imaging system; defining a phantom coordinate system attached to the registration phantom using the acquired at least one 2D X-ray image; determining a pose of the phantom coordinate system in a chosen coordinate system; defining the restricted access volume in the chosen coordinate system to include the region of interest, an envelope of said restricted access volume being computed based on the determined pose of the phantom coordinate system in the chosen coordinate system. The invention also relates to a system implementing this method to treat a region of interest of a patient's body.

## Description

### FIELD OF THE INVENTION

The invention relates to a surgical robotic system and method for defining a restricted access volume for such a surgical robotic system.

### BACKGROUND OF THE INVENTION

There is an increasing use of surgical robotic systems for assisting a user (e.g. a surgeon) during a surgical intervention.

For example, in spine surgery, the user may have to implant one or several screws into at least one vertebra. A robotic arm may assist the user by holding a drill guide and maintaining the drill guide according to a planned axis. The user may thus use a handheld drill passing through the drill guide held by the robotic arm to drill a hole intended to receive the screw in a vertebra along the planned axis.

In this regard, the use of a localization system that can localize trackers in real-time (high frequency, low latency) may be used to carry out such manipulation, either in assistance of the surgeon or autonomously. Both the anatomical structure and the surgical tool can be localized, which allows determining in real time the relative positions of the surgical tool relative to the anatomical structure to be treated.

To that end, both the surgical tool and the anatomical structure may comprise a tracker rigidly attached thereto, each tracker being tracked by the localization system.

In some circumstances, a tracker may be rigidly attached to a part of the surgical robotic system and not the surgical tool directly, allowing indirect localization of the tool based on knowledge at any time of the kinematic model of the surgical robotic system between the tracker and the tool.

However, an autonomous manipulation by a robotic system of a surgical tool for treating an anatomical structure comes with important safety issues. Indeed, after the planning of the trajectory of the surgical tool, the surgeon does not act in a direct manner to execute the planned surgical procedure. Moreover, the tracker attached to the anatomical structure may move, e.g. as a result of an involuntary shock or push applied to it. Or the tracker attached to the surgical tool can be off its calibration. Or any software error can lead to a geometric error leading to a false position of the autonomous robot while it executes its planned trajectory. In a similar fashion, a tracker could be defective for numerous reasons (blood spilled on it in case of optical tracking, electromagnetic disturbances in case of electromagnetic tracking), resulting in erroneous tracking.

It is therefore important to enhance the safety of robotically assisted or robotically conduced surgical procedures by being able to define a prohibited volume into which the surgical system is configured to never enter.

Document WO2018081136 discloses a method for defining such a prohibited volume. In this document, the skin surface of a patient is identified in an image dataset of the patient's anatomy to generate a boundary surface. The image dataset and the boundary surface are then registered within a common coordinate system to define the prohibited volume. However, this method does not work well with all imaging system, particularly with X-ray cone beam computed tomography (CBCT). Indeed, it will be harder to identify the skin surface of the patient using X-ray image acquired with a CBCT imaging system since the grey value of the pixels associated to the patient's skin will have more variation between each X-ray image compared to images acquired with other X-ray imaging system because of the CBCT technology.

There is also a solution which uses a camera disposed on the extremity of a robotic arm to do a visual scan of the patient skin to define the prohibited volume. However, this method requires an additional step and extra material.

A purpose of the invention is to propose a simple solution not requiring extra step or extra material and working for any X-ray imaging system.

### BRIEF DESCRIPTION OF THE INVENTION

According to a first aspect, the invention is directed towards a method for determining a restricted access volume for a surgical robotic system comprising a robotic arm and an end-effector adapted to treat a region of interest of a patient's body, a registration phantom comprising a set of radiopaque fiducials, the registration phantom being located in proximity to the region of interest, the method comprising:
- acquiring at least one 2D X-ray image containing the region of interest and the set of fiducials of the registration phantom by an imaging system;
- defining a phantom coordinate system attached to the registration phantom using the acquired at least one 2D X-ray image;
- determining a pose of the phantom coordinate system in a chosen coordinate system;
- defining the restricted access volume in the chosen coordinate system to include the region of interest, an envelope of said restricted access volume being computed based on the determined pose of the phantom coordinate system in the chosen coordinate system.

Some preferred but non-limiting features of the method described above are the following, taken alone or in any technically feasible combination:
- the phantom coordinate system is defined by first, second and third axes, the method comprising:
   - defining a first axis as rotation axis of the X-ray imaging system (300);
   - using the at least one 2D X-ray image to define an intermediary axis;
   - determining a second axis using the first axis and the intermediary axis;
   - determining a third axis using the first axis and the second axis to generate the phantom coordinate system defined by the first, second and third axes.
- defining the intermediary axis comprises:
   - determining a position of each fiducial using the acquired at least one 2D X-ray image;
   - determining a main plane of the registration phantom using the determined position of each fiducial and a known relationship between the fiducials and a geometry of the registration phantom;
   - defining the intermediary axis as a normal vector to the main plane of the registration phantom.
- the envelope of the restricted access volume is computed to comprise at least part of the main plane of the registration phantom.
- the step of defining the restricted access volume comprises:
   - identifying a highest point of the registration phantom in the phantom coordinate system along the third axis;
   - computing the envelope of the restricted access volume to comprises at least part of limitation plane including the identified highest point of the registration phantom and having a normal vector according to the third axis of the phantom coordinate system.
- the highest point of the registration phantom can either be a fiducial or a particular part of the registration phantom such as a corner of the registration phantom.
- at least one dimension of an operating table on which the patient's body lies and/or at least one dimension of the patient's body is further used to compute the envelope of the restricted access volume.
- dimension and shape of the restricted access volume can be further adjusted manually by a user.
- the restricted access volume comprises at least:
   - a first sub-volume, into which the surgical robotic system can enter occasionally under specific conditions; and
   - a second sub-volume, into which the surgical robotic system is prohibited from entering,
   the first sub-volume is determined by the method described above.
- the secondary sub-volume is defined by using information inputs specific to a planning of the treatment of the region of interest and/or to an anatomy of the patient.
- the information inputs comprise the position of at least one surgical item or anatomical structure, and the second sub-volume comprises a set of geometrical forms attached to each of the at least one surgical item or anatomical structure.

According to a second aspect, the invention is directed towards a system to treat a region of interest of a patient's body, said system comprising:
- a registration phantom configured to be located in proximity of the region of interest, the registration phantom comprising a set of radiopaque fiducials;
- an X-ray imaging system configured to acquire at least one 2D X-ray image containing the region of interest and the set of fiducials;
- a surgical robotic system configured to treat the region of interest of a patient's body, the surgical robotic system comprising a robotic arm, an end-effector mechanically coupled to a distal end of the robotic arm and a control unit, the control unit being configured to:
   * implement the method described above monitor the position of any part of the surgical robotic system in relation with the restricted access volume;
   * restrict or prohibit movement of the robotic arm if the control unit detects that at least a part of the surgical robotic system enters, or is going to enter, the restricted access volume.

Some preferred but non-limiting features of the system described above are the following, taken alone or in any technically feasible combination:
- the control unit is configured to control the movement of the robotic arm, to treat the region of interest, by computing and applying a trajectory of the robotic arm respecting constraints in relation with the restricted access volume.
- the control unit is further configured to:
   - define two sub-volumes to determine the restricted access volume using the method described above;
   - monitor a position of each part of the surgical robotic system to detect if at least a part of the surgical robotic system enters, or is going to enter, at least one of the two sub-volumes;
   - if a part of the surgical robotic system is detected to enter, or going to enter, the first sub-volume, allow the movement of the arm under specific conditions;
   - if a part of the surgical robotic system is detected to enter, or going to enter, the second sub-volume, prohibit the movement of the robotic arm.
- the control unit is configured to compute the trajectory of the robotic arm respecting the following rules:
   - when possible, the trajectory must prevent each part of the surgical robotic system from entering the restricted access volume;
   - the trajectory can at most lead the surgical robotic system to only enter, under specific conditions, the first sub-volume of the restricted access volume;
   - the trajectory must prevent each part of the surgical robotic system from entering the second sub-volume.
- The system comprises:
   - a localization system coupled to the surgical robotic system;
   - a patient tracker, localizable by the localization system, disposed on the patient's body and having a known relationship with the registration phantom,
   wherein, the position of the restricted access volume being known in relation with the position of the patient tracker, when the localization system loses visibility of the patient tracker, the control unit is configured to :
   - consider that the position of the restricted access volume access did not change from last known position; and
   - update the position of the restricted access volume as soon as the visibility of the patient tracker by the localization system is restored.

### BRIEF DESCRIPTION OF THE DRAWINGS

Further features and advantages of the invention will be apparent from the following description, based on the appended drawings wherein:
- FIG. 1 is a general overview of a surgical site including an operating table, a motorized C-arm and a surgical robotic system;
- FIG. 2 schematically illustrates a motorized C-arm, an operating table and a patient's body;
- FIG. 3 schematically illustrates a registration phantom and a patient tracker attached to a bone of the patient;
- FIG. 4A schematically illustrates a first embodiment of the definition of the restricted volume according to the method of the invention;
- FIG. 4B schematically illustrates a second embodiment of the definition of the restricted volume access according to the method of the invention;
- FIG. 5 schematically illustrates an example where the definition of the restricted volume access comprises defining two sub-volumes according to the method of the invention;
- FIG. 6 is a flowchart illustrating the method implemented by the control unit of the surgical robotic system to determine the restricted access volume.

For sake of legibility of the drawings, the figures are not necessarily drawn to scale, in particular the dimension of the restricted access volume are here illustrate as having finite dimension for sake of drawing but this is not necessarily the case.

The reference signs identical from one figure to another one designate the same elements or elements fulfilling the same function.

### DETAILED DESCRIPTION OF EMBODIMENTS

The method may be implemented for operating a robotic system in the context of a surgical intervention carried out onto a patient's bone, including but not limited to: implantation of orthopaedic implants such as pedicular screws in the spine, implantation of various orthopaedic implants in bones, reduction and fixation of fractures during traumatological procedures, positioning guides or canulae at a desired position with respect to a predefined target, or insertion of catheters or stents during cardio-vascular or urology procedures.

In this regard, the surgical robotic system is coupled to an X-ray imaging system and may also be coupled to a localization system.

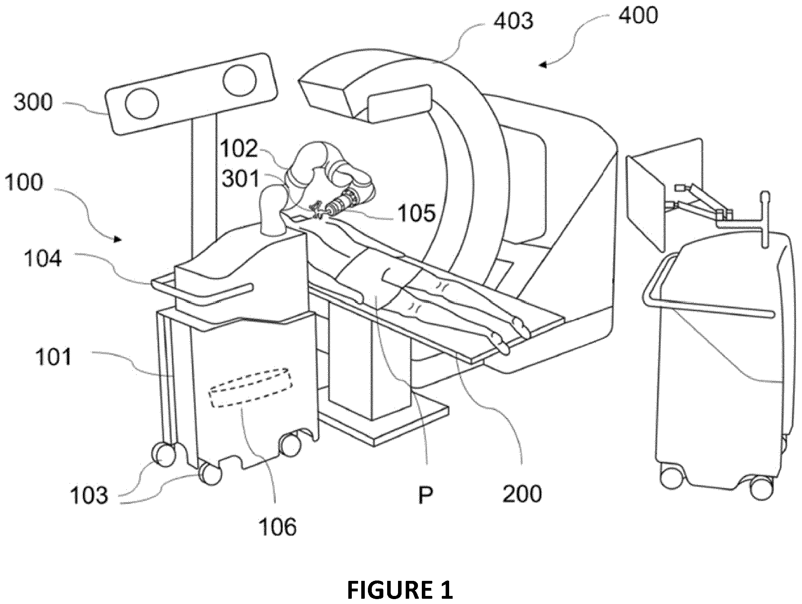

FIG. 1 is a general overview of a surgical site including a surgical robotic system 100, an operating table 200, a localization system 300 and a motorized C-arm 400.

### Surgical robotic system

The surgical robotic system 100 has a movable cart 101 and a robotic arm 102 carried by the movable cart.

During a surgical operation, the cart 101 is intended to remain fixed relative to an operating table 200 on which a patient P lies, while the robotic arm 102 is moved to treat the region of interest R of the patient's body P.

The movable cart 101 may be mobile on wheels 103 and include at least one handle 104 to allow an operator to easily maneuver and transport the surgical robotic system.

The movable cart 101 may be manually actuated or, alternatively, motorized with at least one degree of freedom. At least one of the wheels may be blocked once the movable cart has been moved to the desired position with respect to the operating table.

The robotic arm 102 comprises a plurality of degrees of freedom in translation and/or rotation. Usually, the robotic arm comprises at least six motorized degrees of freedom. To that end, the robotic arm comprises a plurality of articulated segments driven by motors with encoders. By convention, the segments are numbered from the proximal end (which is the end closest to the movable cart) to the distal end (opposite the proximal end) of the robotic arm. The successive robotic arm joints may be rotations or translations. Successive rotations may be orthogonal or parallel. Some parts of the robotic arm may also use parallel mechanisms such as a hexapod architecture.

In preferred embodiments, the robotic arm is made of six or seven rotation axes arranged in an anthropomorphic architecture, such as for example the KUKA liwa^{™} or LBR Med^{™}, STAUBLI Puma 200^{™}, or KINOVA Gen 3^{™}. In such anthropomorphic architectures, the first and second axes are substantially orthogonal to each other.

The robotic arm may be either controlled in an autonomous mode according to desired targets and trajectories, or manipulated using a collaborative mode (cobot), or telemanipulated using a master control device. A combination of two or more of these different modes may be used on the same robotic system.

The surgical robotic system 100 comprises an end-effector 105 for holding a medical device such as a surgical tool or a tool holder, said end-effector being mechanically coupled to the distal end of the robotic arm 102.

The surgical robotic system 100 also includes a control unit 106 configured to controllably move the robotic arm 102. In some embodiments, the control unit is configured to also controllably move at least part of the movable cart 102 (e.g. at least one wheel).

The control unit 106 comprises a processor, a data storage device and a communication device. The control unit may advantageously be embedded in the movable cart 101. The movable cart may also comprise switches, such as power switches, an emergency button or the like.

The control unit 106 is configured to:
- implement the method of determination of the restricted access volume V, that will be described later in the description;
- monitor the position of any part of the surgical robotic system 100 in relation with the restricted access volume V;
- restrict or prohibit the movement of the robotic arm 102 if the control unit detect that any part of the surgical robotic system 100, such as the robotic arm 102 or the end-effector 105, enters, or is going to enter, the restricted access volume V.

When the robotic arm 102 is controlled in an autonomous mode, the control unit 106 is also configured to control the movement of the robotic arm 102, to treat the region of interest R, and compute a trajectory of the robotic arm 102 respecting constraints in relation with the restricted access volume V.

In other embodiments (not illustrated), the control unit 106 may be provided separate from the movable cart 101 and may be configured to communicate wirelessly or by wires with the robotic arm 102.

The control unit 106 may be a single unit controlling the various elements of the system, such as the surgical robotic system 100, the imaging system 400 and the localization system 300, or correspond to a set of units communicating with each other, each unit controlling a respective element of the system. In the following description, the term "control unit 106" is used indifferently to designate one of these cases.

### Localization system

The localization system 300 is configured to localize trackers attached to the patient, the robotic arm, the end-effector and/or the surgical tool. The localization system 300 may be chosen among various technologies, such as optical localization (for example Aurora from NDI, Canada), electromagnetic localization, or ultrasound localization.

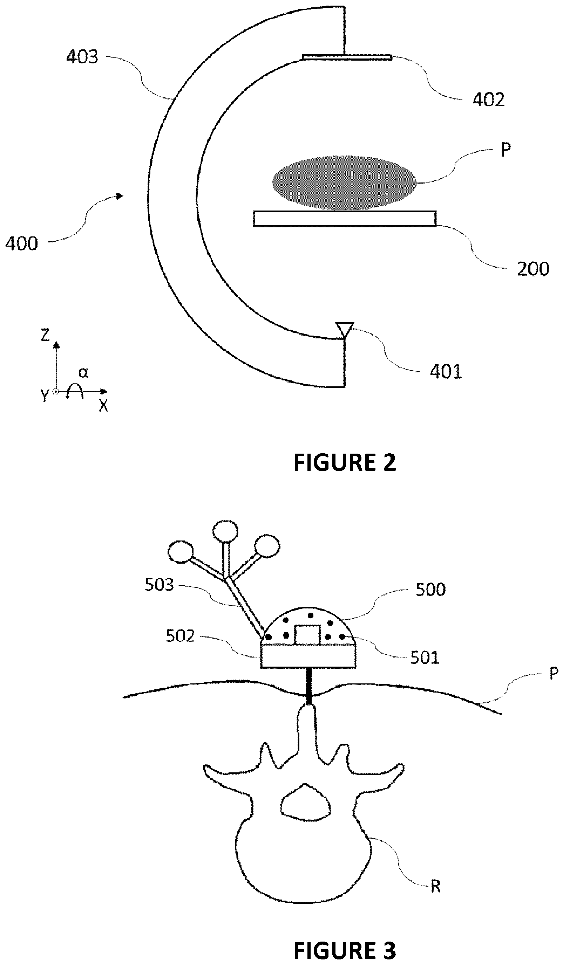

In the embodiment illustrated in FIG. 1, the localization system comprises a camera 300 arranged to detect optical trackers, wherein each optical tracker comprises a set of reflective markers, having for example a spherical shape. In FIG. 1, only one tracker 301 has been represented attached to the end-effector 105 (also called "robot tracker"), but at least one additional tracker 503 attached to the patient (also called "patient tracker") is also used as illustrated in FIGS. 3, 4A, 4B and 5.

### X-ray imaging system

As depicted in more detail in FIG. 2, the X-ray imaging system 400 comprises at least one X-ray source 401 and at least one X-ray image detector 402. The X-ray imaging system produces at least one 2D X-ray image that is the result of a conical projection of a patient anatomy, wherein the tip of the cone is approximately the central point of the X-ray source and the basis of the cone is approximately the portion of the X-ray image detector that is reached by X-ray beams that have been collimated in a given shape and orientation.

For example, the X-ray imaging system can be a conventional C-arm, or any Cone-Beam Computed Tomography (CBCT), such as the Surgivisio device (Surgivisio, Gieres, France), or Vision FD Vario 3D (Ziehm), CIOS Spin Mobile 3D (Siemens), Airo (Stryker), Loop-X (Brainlab), O-arm (Medtronic).

A conventional C-arm is designed to allow the X-ray source and X-ray detector to rotate along a C-shaped gantry 403 while obtaining projection images of the patient placed between the X-ray source and the X-ray detector of the gantry.

A CBCT has a mobile X-ray source and a mobile X-ray image detector, wherein the X-ray source and the X-ray image detector have motorized motions, moving together or independently. A CBCT can have a C-arm shape or an O-arm shape. It can be used to acquire a set of 2D X-ray images over approximately 180° of orbital rotation that can be combined with translations and from which a 3D image can be reconstructed using tomography algorithms or tomosynthesis algorithms.

The X-ray imaging system may be motorized, notably the C-shaped arm may comprise motors allowing movement horizontally (X and Y direction), vertically (Z direction) and around the X direction (defined by an angle α), so that 2D X-ray images of the patient are produced from almost any angle. As shown on FIG. 2, the X axis is transversal to the operating table 200 on which the patient lies and the Y axis, also called orbital rotation axis, is parallel to the longitudinal axis of said operating table. Each motor is associated to an encoder that provides at any time the relative position of the medical imaging system with respect to a reference position. When a 2D X-ray image is acquired, the corresponding position of the imaging system is recorded. Thus, each 2D image is recorded in the referential of the imaging system.

In some embodiments, a 3D image of the patient may be obtained during the surgery, using the X-ray imaging system itself if it is a CBCT. Said 3D image is registered with respect to a tracker, and/or a registration phantom, attached to the anatomical structure using known methods of calibration and navigation.

In other embodiments, the 3D image may be acquired prior to surgery using a Computed Tomography (CT) device or another CBCT device. Said 3D image of the patient is registered with respect to the tracker, and/or the registration phantom, attached to the anatomical structure using a 3D registration method that can use many techniques, such as (i) a collection of surface points using the localizing system and their fitting on the anatomical structure like in the technique provided by 7D Surgical (North York, Canada), or (ii) the acquisition of 2D X-ray images calibrated with respect to the registration phantom attached to the anatomical structure and used for registration with the 3D image like in the technique provided in the Mazor X robotic system (Medtronic), or (iii) any registration technique using localized ultrasound images, fiducials, anatomical points and the like.

### Registration phantom

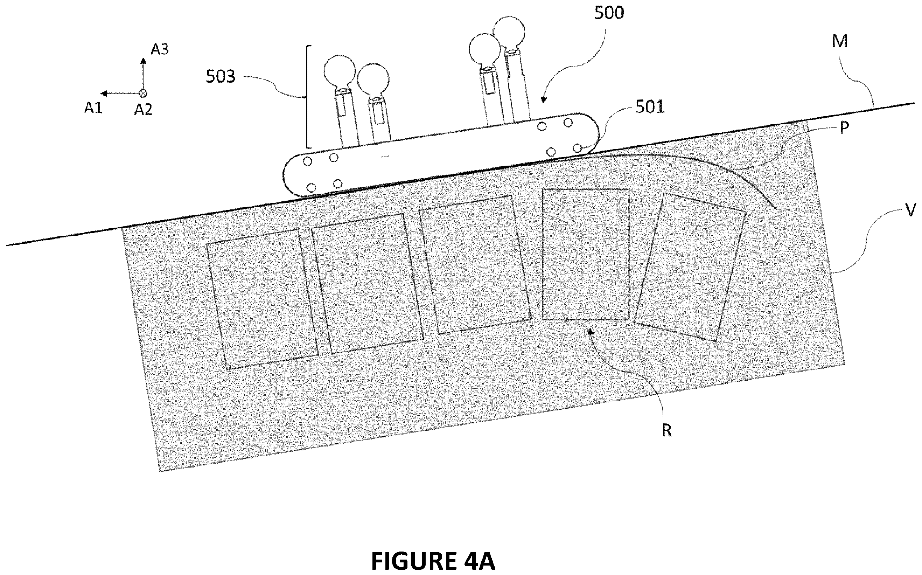

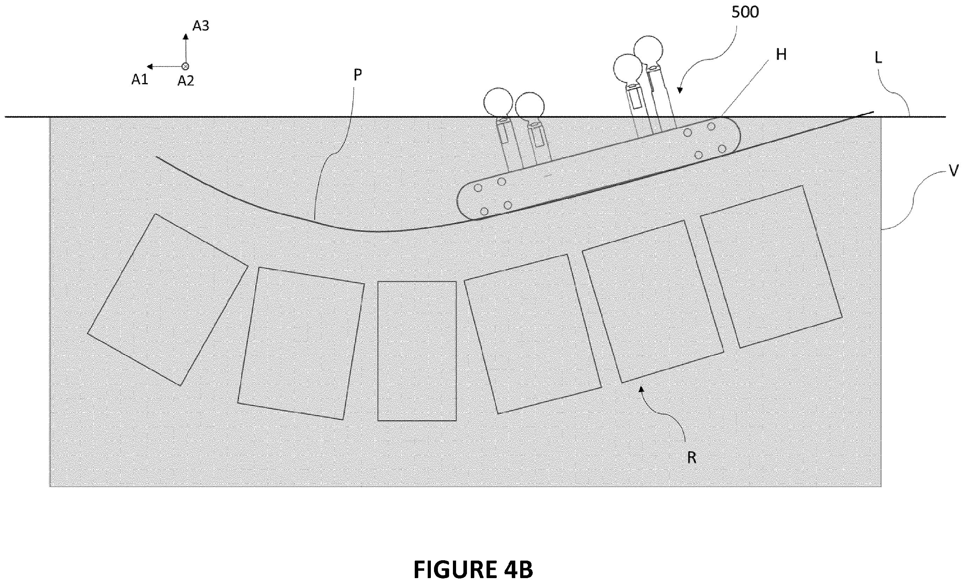

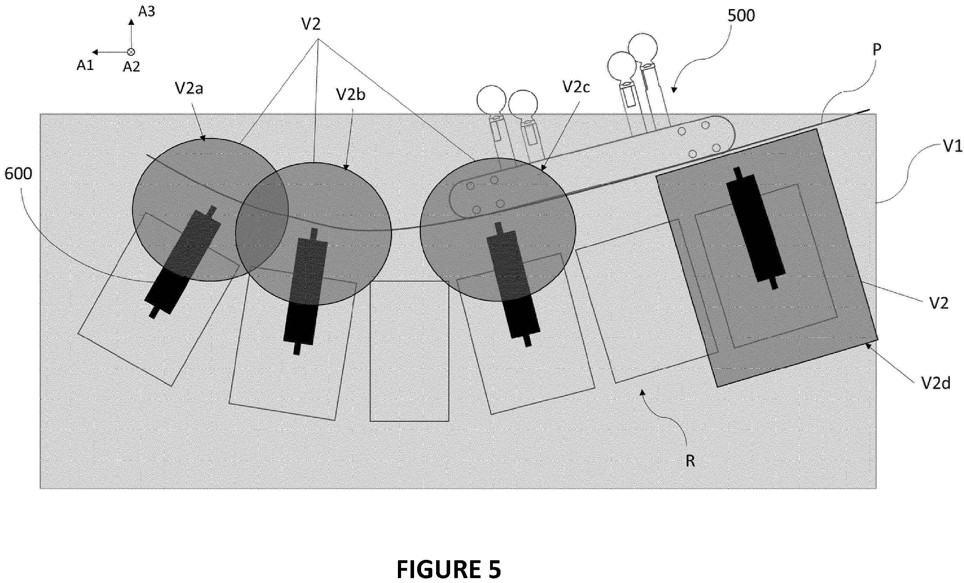

At the beginning of a surgical intervention, a registration phantom 500 is placed onto the patient in proximity of the region of interest R to be treated such as a bone. Preferably, the placement of the registration phantom 500 is done without any surgical step, for example by using an adhesive to attach the registration phantom to the patient's skin (as illustrated in FIGS 4A, AB and 5) or by attaching the registration phantom to a device already implanted into a patient's bone R for another purpose, such as the base 502 intended to attach a patient tracker 503 to the bone (as illustrated in FIG. 3). Optionally, as illustrated in FIGS 4A, 4B and 5, the registration phantom 500 can include a tracker 503. This tracker 503 can be the patient tracker, or an additional tracker called "phantom tracker".

The registration phantom 500 comprises a set of radiopaque fiducials 501, in particular three or more radiopaque fiducials. Each fiducial 501 has a known position in a 3D coordinate system of the registration phantom and so has a known relationship with the geometry of the registration phantom 500.

Each fiducial 501 may be a ball-shaped or a needle-shaped piece of radiopaque material, such as steel, stainless steel, or zircon, and whose dimensions are known by any methods available to the skilled person. The fiducials 501 are embedded in or supported by a support made of a substantially radiotransparent material. In this way, when a 2D X-ray image of the registration phantom is acquired, the fiducials 501 are visible in the image whereas the support is substantially not visible, or at least does not hinder the detection of the fiducials in the 2D X-ray image.

The registration phantom 500 is here used to generate the 3D image from the 2D X-ray images acquired by the imaging system, as described before, and to define the restricted access volume. Indeed, since the registration phantom 500 is generally in close proximity to the patient's skin, it can be assumed that the registration phantom position and orientation are strongly linked to the patient's skin surface properties (such as position and orientation) in the vicinity of the region of interest R to be treated.

It is then possible to define a restricted access volume V using the information of the registration phantom 500 without needing extra material, such as an additional sensor or acquisition device, since the registration phantom 500 is already present on the patient as part of the generation of the 3D image.

### Restricted access volume

The restricted access volume V is a volume into which the surgical robotic system 100 is configured to only have a restricted access. In other word, any part of the surgical robotic system 100 (robotic arm 102, end-effector 105, and even the surgical tool coupled to the end-effector if its dimensions are known by the surgical robotic system) is forbidden to enter this volume, excepted under specific conditions. For example, the surgical robotic system 100 may be allowed to enter the restricted access volume V if only a part of the surgical robotic system 100 enters the restricted access volume while the end-effector and the instrument never enter the restricted access volume. Indeed, to properly position the end-effector to treat the region of interest, only a part of a segment of the robotic arm may temporarily enter the restricted access volume while not endangering the patient's life. In this particular case, there is no reason to forbid the movement of the robotic arm. In another example, the end-effector can be equipped with a tool holder and, depending on the operation, it could be needed to allow the tool holder to enter the restricted access volume V, in specific areas, while the rest of the surgical robotic system 100 stays out of the restricted access volume V. Part of the surgical robotic system 100 could also only be allowed to enter the restricted volume V, in specific areas, at specific step of the planned surgical procedure.

The restricted access volume V is chosen to enclose the region of interest R, and at least part of the patient's body P, to reduce risks of injury of the patient by the robotic arm 102, in particular during displacement of the robotic arm 102 to guide the surgical tool according to a planned trajectory.

Advantageously, the restricted access volume V comprises at least two sub-volumes each sub-volume defining a different security level with different access conditions:
- a first sub-volume V1, into which the surgical robotic system 100 can enter occasionally under specific conditions;
- a second sub-volume V2 into which the surgical robotic system 100 is prohibited from entering. This second sub-volume V2 is generally linked to vulnerable and vital part of the patient and any contact between this second sub-volume V2 and the surgical robotic system 100 could lead to irreversible damage to the patient.

In this embodiment, the surgical robotic system 100 can be allowed to enter the restricted access volume V only if it enters temporarily the first sub-volume V1 while never crossing the second sub-volume V2. Or the surgical robotic system 100 can be allowed to enter the restricted access volume V only if specific parts of the robotic system 100 enter the first sub-volume V1 while no part of the surgical robotic system 100 crosses the second sub-volume V2. The second sub-volume V2 may substantially correspond to the patient's spine, including all vertebrae, spinal cord and nerve entry point in said spinal cord (together with defined as a region of interest). In another embodiment, the second sub-volume V2 may instead, or additionally, take into account the information input by the surgeon during the planning of the surgical procedure on the 2D and/or 3D images acquired preoperatively, such as the position of at least one surgical item 600 to be inserted into the region of interest R as illustrated in FIG. 5. The second sub-volume V2 can be defined manually or automatically.

The second sub-volume V2 can be included, or partially included, in the first sub-volume V1. Advantageously, the first sub-volume V1 corresponds to a first delimitation of the restricted volume V while the second sub-volume V2 is used to add an extra security layer by defining at least one area where any movement is forbidden. When the second sub-volume V2 includes area not comprised in the first sub-volume V1, and so the initial restricted access volume V, the second sub-volume V2 is used to update the restricted access volume V. In this case, the restricted access volume V corresponds to the union of the first and second sub-volumes V1, V2.

Each sub-volume V1,V2 may comprises several distinct volumes as illustrated in FIG. 5 where the second sub-volume V2 comprises four volumes V2a,V2b,V2c,V2d, each of those volume being attached on a surgical item to be inserted into the region of interest R.

Obviously, the restricted access volume V can comprise more than two sub-volumes.

In addition, some specifics volume could be excluded from the restricted access volume V. Indeed, in the case of pedicle screw placement, the surgeon may indicate a drilling axis that the robotic arm 102 will have to maintain for the drill bit through the positioning of each pedicle screw. As the robotic arm 102 and the tool held or guided by the robotic arm 102 may have to evolve at least partially near these axes during drilling, it may be advantageous to exclude volumes, such as cylinders or cones whose axis of revolution correspond to the planned drilling axis, around said axes from the restricted access volume.

### Region of interest

The region of interest R to be treated is usually a bone (or a plurality of bones as illustrated in FIG.4A, 4B and 5) that may be drilled, burred, and/or milled in order to place an implant or to free some space for any clinical reason.

### Method for defining the restricted access volume

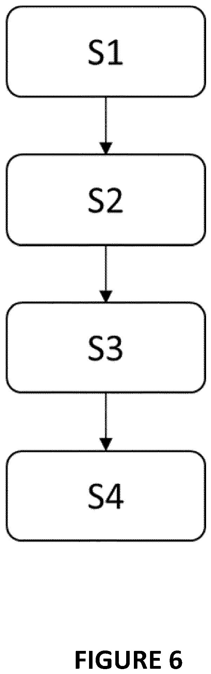

FIG. 6 is a flowchart illustrating the method implemented by the control unit 106 of the surgical robotic system 100 to determine the restricted access volume V. This flowchart is a non-limitative example and some steps may be omitted or carried out in a different order if appropriate.

The method for determining a restricted access volume V comprises the following steps:
- Step S1: acquiring at least one 2D X-ray image containing the region of interest R and the set of fiducials of the registration phantom 500 by the imaging system 400;
- Step S2: defining a phantom coordinate system attached to the registration phantom using the acquired at least one 2D X-ray image;
- Step S3: determining a pose of the phantom coordinate system in a chosen coordinate system;
- Step S4: defining the restricted access volume, in the chosen coordinate system, to enclose the region of interest, and computing an envelope of the restricted access volume based on the determined pose of the phantom coordinate system in the chosen coordinate system.

In step S1, the imaging system 400 acquires at least one 2D X-ray image containing the region of interest R and the set of fiducials 501 of the registration phantom 500. In one embodiment, this step corresponds to the acquisition of 2D X-ray images to generate the 3D image of the region of interest used by the surgeon to do the planning of the operation. As it is well known by the skilled person, a minimum of three fiducials are required to be visible in the 2D X-ray image to determine the pose of the phantom coordinate system. It is not required to see the full set of fiducials, but the more fiducials are visible, the lower the risk of error.

In step S2, the phantom coordinate system attached to the registration phantom is defined. First, the position of each fiducial is determined in the coordinate system of the imaging system, or of the 3D image if a 3D volume is reconstructed, using the at least one 2D X-ray image.

In one embodiment, If the projection matrix of the imaging system 400 is known, it is possible to determine to the position of each fiducial in coordinate system of the imaging system with only one or two 2D X-rays images, without needing to reconstruct a 3D volume, using back-projection and the known relationship between each fiducial. The projection matrix considers the geometric model of the X-ray imaging system and defines the transformation of a 3D point of the coordinate system of the imaging system to a 2D-pixel of a 2D X-ray image. This projection matrix can be provided by the manufacturer of the X-ray imaging system or estimated with a calibration method. The more 2D X-ray images available with sufficiently different X-rays angle of incidence, the more accurate the localization of each fiducial will be.

In another embodiment, the projection matrix of the imaging system is not available and a plurality of 2D X-rays images, with preferentially sufficiently different X-rays angle of incidence, are required to construct a 3D image using well known techniques. Once the 3D image is generated, the position of the fiducials in the coordinate system of the imaging system, or of the 3D image, is obtained by detecting those fiducials in the 3D image.

Alternatively, a 3D volume of the region of interest could have been acquired preoperatively with another imaging system and/or without the registration phantom visible in this 3D volume. In this case, at least one 2D X-ray image can be acquired by the imaging system and then registered with the 3D volume, using well known technique such as using correlation metrics or finding correspondence between image features, to obtain the position of the fiducials in the 3D volume.

Then, the main plane M of the registration phantom is determined using the determined position of each fiducial and the known relationship between the fiducials and the geometry of the registration phantom. This main plane M can be a theorical plane representing the main shape of the registration phantom or could correspond to a particular plane of the registration phantom. In particular, this plane could be the contact surface of the registration phantom with the patient's skin, if the registration phantom is attached to the patient's skin with an adhesive, or the contact surface with a device already implanted into a patient's bone, such as the base 502 on which the patient tracker 503 is intended to be attached, if the registration phantom 500 is not directly in contact with the patient's skin.

Once the main plane M of the registration phantom 500 is determined, an intermediary axis is defined as the normal vector to this main plane. This intermediary axis is used to define the phantom coordinate system as described below.

To define the phantom coordinate system, three axes are required. The first axis A1 is defined as the rotation axis Y of the X-ray imaging system 300 used to acquire the at least one X-ray image previously used to determine the position of each fiducial. This first axis can also, alternatively, be associated to the cranio-caudal axis of the patient or correspond to the orbital rotation axis Y of the imaging system used to acquire preoperatively the 3D volume in the case of a 3D volume being available preoperatively as mentioned before. Then, the second axis A2 is determined using the first axis and the intermediary axis previously determined by using a vector product. Finally, the third axis A3 is determined, similarly to the second axis, using the first and second axis. The first, second and third axes A1, A2, A3 thus obtained form the phantom coordinate system.

In step S3, the pose of the phantom coordinate system is determined in a chosen coordinate system.

In one embodiment, if the localization system 300 has a fixed position during the full duration of the surgery, the chosen coordinate system is the coordinate system of the localization system 300 which can localize both the patient tracker 503 and robot tracker 301. To determine the pose of the phantom coordinate system in the coordinate system of the localization system there is a need to have a known relationship between the registration phantom 500 and an element localizable by the localization system 300. Indeed, the localization system 300 can have access to the position of this element in the coordinate system of the localization system and the control unit 106, knowing the relationship between this element and the registration phantom 500, can then determine the pose of the phantom coordinate system in the coordinate system of the localization system. In a first example, this element is a tracker 503 having a known relationship with the registration phantom 500. This tracker 503 can be directly integrated in the registration phantom 500 (as illustrated in FIGS. 4A, 4B and 5) and can correspond to the patient tracker or to an additional tracker, different from the patient tracker, called "phantom tracker". Alternatively, this tracker 503 can have a known mechanical liaison with the registration phantom 500, like both sharing a common base (as illustrated in FIG. 3). In a second example, this element is a tracker attached to the imaging system. In this case, the relationship between the registration phantom 500 and the imaging system 400 is known through the detection of the position of the registration phantom in the 2D X-ray image, or directly in the 3D image reconstructed from the 2D X-ray images, using image processing techniques.

In a preferred embodiment, the chosen coordinate system is the coordinate system attached to the patient tracker, also called "patient coordinate system". This embodiment is advantageous since the patient tracker 503 is supposed to have a fixed position in relation with the region of interest R during the full duration of the operation. The pose of the phantom coordinate system is then directly obtained through a known relationship between the registration phantom 500 and the patient tracker 503.

In a first embodiment, the patient tracker 503 is directly integrated in the registration phantom 500 (as illustrated in FIGS. 4A, 4B and 5) or has a known mechanical link with the registration phantom 500, like both sharing a common base (as illustrated in FIG. 3).

In a second embodiment, the registration phantom 500 is equipped with its own tracker called "phantom tracker", different from the patient tracker. In this case the relationship between the registration phantom 500 and the patient tracker is directly obtained through the localization system 300 which can both localize the patient tracker and the phantom tracker.

In the following description, the patient coordinate system is selected as the chosen coordinate system. Obviously, any other relevant coordinate system can be selected, and other methods may exist and be used to determine the pose of the phantom coordinate system in the chosen coordinate system.

In step S4, the restricted access volume V is defined, in the patient coordinate system, using the determined pose of the phantom coordinate system. More specifically, the envelope of the restricted access volume is computed based on the pose of the phantom coordinate system. In addition, the restricted access volume includes the region of interest. As mentioned before, if there is a need to define the restricted access volume V in a different chosen coordinate system, the pose of the phantom coordinate system is determined in this chosen coordinate system instead of the patient coordinate system.

In a first embodiment, the restricted access volume V is defined as the volume under the main plane M of the registration phantom 500 (assuming that the registration phantom is located above the patient's body as illustrated in FIG. 4A). Indeed, since the registration phantom 500 is generally in close proximity to the patient's skin, the main plane of the registration phantom 500 can be associated to the surface of the patient's skin. As a result, the envelope of the restricted access volume comprises at least part of the main plane M.

However, this definition may not be always appropriate, in particular when the region of interest R is a vertebra and that the patient has a significant spinal deformity such as kyphosis or scoliosis as illustrated in FIG 4B.

In a second embodiment, particularly adapted when the patient has a spinal deformity, the restricted access volume V is defined by using the "highest point" H of the registration phantom 500. This highest point H of the registration phantom 500 may correspond to a fiducial 501 or to a particular part of the registration phantom, such as a corner, which is the highest point in the phantom coordinate system along the third axis. Once this highest point H is determined, the restricted access volume V is defined as the volume under the limitation plane L comprising the identified highest point H, the limitation plane L being orthogonal to the 3rd axis of the phantom coordinate system. In other words, the limitation plane L is parallel to the plane formed by the first and second axes of the phantom coordinate system and has the third axis of the phantom coordinate system as its normal vector. This limitation plane is used as an extra security to reduce the risk of excluding important patient's body part from the restricted access volume V when the surface around the region of interest presents a local deformity compared to the surrounding surface. As a result, the envelope of the restricted access volume comprises at least part of the limitation plane L.

Additionally, for both embodiments, at least one dimension of the operating table 200 and/or at least one dimension of the patient's body P can be used to further delimit the restricted access volume V and thus compute the envelope of the volume V. Indeed, the patient's body P does not normally protrude from the operating table 200 so there is no need to extend the dimension of the restricted access volume V outside the border of the operating table 200. When the patient P lies on the operating table 200 on his side, in lateral decubitus position with the registration phantom 500 attached to a vertebra, it is advantageous to take into account the patient's width to define a top border of the restricted access volume V to allow movement of the surgical robotic system 100 on top of the patient body P. In addition, the restricted access volume V does not need to include the full patient's body P, since the robotic arm 102 may not be able to reach parts of the patient's body P that are far from the region of interest and/or these parts are less prone to injuries.

In one embodiment, the restricted access volume V could have an ellipsoid shape such as a cylinder, the cylinder being extruded from the initial shape of the restricted access volume V. In this example, the cylinder can be defined such as a generatrix of the cylinder is tangent to the main plane M of the phantom or to the limitation plane L. The dimension of the patient and/or the table are advantageously used to define the properties of the base of the cylinder.

In one other embodiment, dimension and shape of the restricted access volume V can be further adjusted manually by a user. For example, the current restricted access volume V can be displayed on a screen as a layer on top of 3D model of the patient's body or region of interest, and the user could directly adjust the outline of the restricted access volume V.

Advantageously, as previously described, the restricted access volume V comprises at least two sub-volumes (as illustrated in FIG. 5). The first sub-volume V1 is defined using the method previously described and the second sub-volume V2 is defined using information inputs specific to the planning of the treatment of the region of interest R and/or to the anatomy of the patient. Those information inputs may comprise the position of a least one anatomical structure, such as a bone or the region of interest, and/or the position of at least one surgical item 600 such as a pin, screw or implant. The second sub-volume V2 comprises a set of geometrical form attached to each of the at least one surgical item 600 and/or anatomical structure. The geometrical form is advantageously a simple shape for sake of simplicity such as a cube, a sphere or a rectangle, but other more complicated forms can also be chosen if more adapted to the nature of the surgical item 600. The set of geometrical forms does not require to share the same shape and properties, a different geometrical form could be chosen for each different surgical item 600; and/or anatomical structure, with its dimensions adjusted depending on the dimensions of the associated surgical item and/or anatomical structure.

Obviously more than two sub-volumes can be defined for the restricted access volume V, in particular if there is a need for the surgical robotic system 100 to have more than two different behaviors in relation with different parts of the restricted access volume V.

As a reminder, the restricted access volume V corresponds to the union of all the sub-volumes. The shape of the restricted access volume V can change in relation with the sub-volumes, in particular when the second sub-volume V2, or an additional sub-volume, comprises areas not included, or only partially included, in the first sub-volume V1. It is so possible than some areas are only comprised in the second sub-volume V2 while some areas are comprised in both first and second sub-volume V1, V2.

### Operation of the robotic surgical system

During a surgical intervention, the patient P lies on the operation table 200 and a registration phantom 500 is rigidly attached to the patient P, in proximity to the region of interest R.

For example, in case of spine surgery, the patient P may lie face down on the operating table 200, such that the region of interest R, which may comprise one or several vertebrae, is accessible to the surgeon. In another example, not illustrated here, the patient P may lie on his side, in lateral decubitus position, on the operating table 200 for operation such as oblique lateral interbody fusion to have a better access to the lumbar spine.

At the beginning of surgery, the patient P is equipped with a first tracker (called "patient tracker" 503) localizable by the localization system 300 and a registration phantom 500. The patient tracker 503 has a known relationship with the registration phantom 500, and so knowing the position of the patient tracker 503 allow to know the position of the registration phantom 500.

A second tracker 301 (called "robot tracker" 301) can also be mounted on the robotic arm 102, or any sub-system of the surgical robotic system 100. The relationship between the robot tracker 301 and the surgical robotic system 100 is known, the robot tracker 301 having a known position on the surgical robotic system 100. This robot tracker 301 is also localizable by the localization system 300 and allow the control unit 106 to know the position of any part of the surgical robotic system 100 relative to the restricted access volume V. This is possible using the information from the localization system 300, the relationship between the robot tracker 301 and the surgical robotic system 100, and the encoders value of each motor of the robotic arm 102. If the position of the base of the surgical robotic system 100 is known and do not change during the treatment of the region of interest, the robot tracker 301 may not be required.

A 3D image is acquired either at the beginning of the surgery using the X-ray imaging system itself (CBCT) or prior to the surgery with another imaging system (CT or CBCT). In the latter case at least one 2D X-ray image comprised the fiducials of the registration phantom must be acquired as described before if the 3D image obtained prior to the surgery does not comprises the fiducials of the registration phantom.

The control unit 106 implements the previously described method to determine the restricted access volume V. The position of the restricted access volume V is thus known by the control unit 106 in relation with the position of the patient tracker 503 by the localization system, in the patient coordinate system, and so is updated with any movement of the patient tracker 503, such as the patient's breathing.

Then the control unit 106 monitors the position of any part of the surgical robotic system 100 in relation with the restricted access volume V, and restricts or prohibits the movement of the robotic arm 102 if the control unit 106 detects that any part of the surgical robotic system 100 enters, or is going to enter, the restricted access volume V.

When the robotic arm 102 is moved automatically by the control unit 106, the control unit 106 is configured to control the movement of the robotic arm 102, to properly position the end-effector to treat the region of interest R, by computing and applying a trajectory of the robotic arm respecting constraints in relation with the restricted access volume V. To compute the trajectory of the robotic arm 102, the control unit 106 is configured to respect the following rules:
- When possible, the trajectory should avoid any part of the surgical robotic system 100 from entering the restricted access volume V;
- The trajectory can at most lead the robotic surgical system 100 to only enter, under specific conditions such as temporarily or/and partially, the first sub-volume V1 of the restricted volume V;
- The trajectory must avoid any part of the surgical robotic system 100 from entering the second sub-volume V2.

If the control unit 106 fail to find a trajectory respecting those rules, the control unit 106 prohibits the movement of the robotic arm 102.

When the robotic arm 102 is moved manually (cobot), to properly position the end-effector 105 to treat the region of interest R, the control unit 106 monitors the position of any part of the surgical robotic system 100 during the full duration of the operation. If the control unit 106 detects that any part of the surgical robotic system 100 enter the restricted access volume V the control unit 106 restricts or prohibits the movement of the robotic arm 102. If the surgical robotic system 100 is detected to enter the first sub-volume V1, the control unit 106 may allow the movement of the robotic arm 102 under specific conditions as described before, but if the surgical robotic system 100 is detected to enter the second sub-volume V2, the control unit prohibits the movement of the robotic arm. Optionally, this monitoring can also be used when the robotic arm 102 is moved automatically by the control unit 106, as an additional security in case the movement of the robotic arm 102 does not respect the computed trajectory.

In some embodiments, the control unit 106 is further configured to generate a warning signal if any part of the surgical robotic system 100 is at a distance from the restricted access volume V, or any sub-volume V1,V2 of the restricted access volume V, smaller than a predetermined distance such as 30mm. Advantageously, the control unit 106 could generate different warnings, each warning being associated to a specific sub-volume. Those warning could be of various nature such as sound signal, a led changing colour or flashing etc.

If the robotic arm 102 is moved manually, the control unit 106 could send a first warning signal to the user that the surgical robotic system 100 is going to enter the first sub-volume V1 of the restricted access volume V. The user is then invited to try a new trajectory to position the robotic arm 102. If the user does not manage to find such a trajectory, he can be allowed by the control unit 106 to enter the first sub-volume V1 with part of the surgical robotic 100 while none of the part of the surgical robotic system 100 enter the second sub-volume V2. A second warning will be generated if any part of the surgical robotic system 100 is moved to a distance from the second sub-volume V2 lower than the predetermined distance.

If the localization system 300 lost the visibility of the patient tracker 503, and so the position of the restricted access volume V, the control unit 106 is configured to:
- Consider that the position of the restricted access volume access V did not change from last known position;
- Update the position of the restricted access volume V as soon as the visibility of the patient tracker 503 by the localization system 300 is restored.

If the localization system 300 lost the visibility of the patient tracker 503 for a period of time greater than a predetermined value, the control unit 106 can be configured to send a warning and/or to interrupt the movement of the robotic arm 102. Indeed, there is a risk that the patient tracker 503 has been damaged or detached from the patient P which required an intervention from the user before continuing the treatment.

In the above-described method, a patient tracker is needed but it is also possible to apply the method without a patient tracker or even a robot tracker. For example, if the robotic arm holds the registration phantom, and the base of the robotic arm is fixed in relation to the region of interest, there is no need to either use a patient tracker or a robot tracker. The pose of the phantom coordinate system can be obtained directly in the coordinate system of the robotic arm knowing the kinematic of the robotic arm and the relationship between the phantom and the robotic arm.

As a reminder, in the above-described method, the coordinate system used to define the restricted access volume is the coordinate system attached to the patient tracker but the skilled person can choose another coordinate system.

## Claims

1. Method for determining a restricted access volume (V) for a surgical robotic system (100) comprising a robotic arm (102) and an end-effector (105) adapted to treat a region of interest (R) of a patient's body (P), a registration phantom (500) comprising a set of radiopaque fiducials (501), the registration phantom (500) being located in proximity to the region of interest (R), the method comprising:
- acquiring at least one 2D X-ray image containing the region of interest (R) and the set of fiducials (501) of the registration phantom (500) by an imaging system (300) ;
- defining a phantom coordinate system attached to the registration phantom (500) using the acquired at least one 2D X-ray image ;
- determining a pose of the phantom coordinate system in a chosen coordinate system ;
- defining the restricted access volume (V) in the chosen coordinate system to include the region of interest, an envelope of said restricted access volume (V) being computed based on the determined pose of the phantom coordinate system in the chosen coordinate system.

2. Method for determining a restricted access volume (V) according to claim 1, wherein the phantom coordinate system is defined by first, second and third axes, the method comprising:
- defining a first axis (A1) as an orbital rotation axis (Y) of the X-ray imaging system (300);
- using the at least one 2D X-ray image to define an intermediary axis;
- determining a second axis (A2) using the first axis (A1) and the intermediary axis;
- determining a third axis (A3) using the first axis and the second axis to generate the phantom coordinate system defined by the first, second and third axes (A1, A2, A3).

3. Method for determining a restricted access volume (V) according to claim 2, wherein defining the intermediary axis comprises:
- determining a position of each fiducial (501) using the acquired at least one 2D X-ray image ;
- determining a main plane (M) of the registration phantom (500) using the determined position of each fiducial and a known relationship between the fiducials (501) and a geometry of the registration phantom (500) ;
- defining the intermediary axis as a normal vector to the main plane (M) of the registration phantom (500).

4. Method for determining a restricted access volume (V) according to claim 3, wherein the envelope of the restricted access volume is computed to comprise at least part of the main plane (M) of the registration phantom.

5. Method for determining a restricted access volume (V) according to any of claims 2 to 3 wherein the step of defining the restricted access volume comprises:
- identifying a highest point (H) of the registration phantom (500) in the phantom coordinate system along the third axis ;
- computing the envelope of the restricted access volume to comprises at least part of limitation plane including the identified highest point of the registration phantom and having a normal vector according to the third axis of the phantom coordinate system.

6. Method for determining a restricted access volume (V) according to any of claims 4 to 5, wherein at least one dimension of an operating table (200) on which the patient's body (P) lies and/or at least one dimension of the patient's body is further used to compute the envelope of the restricted access volume (V).

7. Method for determining a restricted access volume (V) according to any of claims 4 to 6, further comprising adjusting dimension and shape of the restricted access volume (V) manually by a user.

8. Method for determining a restricted access volume (V) comprising at least:
- a first sub-volume (V1), into which the surgical robotic system (100) can enter occasionally under specific conditions; and
- a second sub-volume (V2), into which the surgical robotic system (100) is prohibited from entering,
wherein the first sub-volume (V1) is determined by the method according to any of claims 1 to 7.

9. Method according to claim 8, wherein the secondary sub-volume (V2) is defined by using information inputs specific to a planning of the treatment of the region of interest (R) and/or to an anatomy of the patient.

10. Method according to claim 9 wherein the information inputs comprise the position of at least one surgical item (600) or anatomical structure, and the second sub-volume (V2) comprises a set of geometrical forms attached to each of the at least one surgical item (600) or anatomical structure.

11. System to treat a region of interest (R) of a patient's body (P), said system comprising:
- a registration phantom (500) configured to be located in proximity of the region of interest (R), the registration phantom (500) comprising a set of radiopaque fiducials (501);
- an X-ray imaging system (300) configured to acquire at least one 2D X-ray image containing the region of interest (R) and the set of fiducials (501);
- a surgical robotic system (100) configured to treat the region of interest (R) of a patient's body (P), the surgical robotic system (100) comprising a robotic arm (102), an end-effector (105) mechanically coupled to a distal end of the robotic arm (102) and a control unit (106), the control unit (106) being configured to:
* implement the method of any of claims 1 to 10 to determine a restricted access volume (V);
* monitor the position of each part of the surgical robotic system (100) in relation with the restricted access volume (V);
* restrict or prohibit movement of the robotic arm (102) if the control unit (106) detects that at least a part of the surgical robotic system (100) enters, or is going to enter, the restricted access volume (V).

12. System according to claim 11, wherein the control unit (106) is configured to control the movement of the robotic arm (102), to treat the region of interest (R), by computing, and applying, a trajectory of the robotic arm respecting constraints in relation with the restricted access volume (V).

13. System according to any of claims 11 to 12, wherein the control unit (106) is further configured to:
- define two sub-volumes (V1,V2) to determine the restricted access volume (V) using the method of any of claims 8 to 10;
- monitor a position of each part of the surgical robotic system (100) to detect if at least a part of the surgical robotic system (100) enters, or is going to enter, at least one of the two sub-volumes (V1, V2);
- if a part of the surgical robotic system (100) is detected to enter, or going to enter, the first sub-volume (V1), allow the movement of the arm under specific conditions;
- if a part of the surgical robotic system (100) is detected to enter, or going to enter, the second sub-volume (V2), prohibit the movement of the robotic arm (102).

14. System according to any of claims 11 to 13, wherein the control unit (106) is configured to compute the trajectory of the robotic arm (102) respecting the following rules:
- when possible, the trajectory must prevent each part of the surgical robotic system (100) from entering the restricted access volume (V);
- the trajectory can at most lead the surgical robotic system (100) to only enter, under specific conditions, the first sub-volume (V1) of the restricted access volume (V);
- the trajectory must prevent each part of the surgical robotic system (100) from entering the second sub-volume (V2).

15. System according to any of claims 11 to 14, further comprising:
- a localization system (300) coupled to the surgical robotic system (100);
- a patient tracker (503), localizable by the localization system (300), disposed on the patient's body (P) and having a known relationship with the registration phantom (500),
wherein, the position of the restricted access volume (V) being known in relation with the position of the patient tracker (503), when the localization system (300) loses visibility of the patient tracker (503), the control unit (106) is configured to :
- consider that the position of the restricted access volume access (V) did not change from last known position; and
- update the position of the restricted access volume (V) as soon as the visibility of the patient tracker (503) by the localization system (300) is restored.
